(19) **Europäisches Patentamt / European Patent Office / Office européen des brevets**

(11) **EP 3 823 588 B1**

(12) **FASCICULE DE BREVET EUROPEEN**

(45) Date de publication et mention de la délivrance du brevet:
**23.10.2024 Bulletin 2024/43**

(21) Numéro de dépôt: **19742363.5**

(22) Date de dépôt: **19.07.2019**

(51) Classification Internationale des Brevets (IPC):
**A61K 9/00** *(2006.01)*  **A61K 9/14** *(2006.01)*
**A61K 31/7048** *(2006.01)*  **A61P 9/14** *(2006.01)*
**A61K 47/26** *(2006.01)*

(52) Classification Coopérative des Brevets (CPC):
**A61K 31/7048; A61K 9/0056; A61K 9/14; A61K 47/26; A61P 9/14**

(86) Numéro de dépôt international:
**PCT/EP2019/069498**

(87) Numéro de publication internationale:
**WO 2020/016408 (23.01.2020 Gazette 2020/04)**

(54) **COMPOSITION PHARMACEUTIQUE SOUS LA FORME D'UN COMPRIME A CROQUER DE DIOSMINE OU D'UNE FRACTION FLAVONOIQUE LA COMPRENANT**

PHARMAZEUTISCHE ZUSAMMENSETZUNG IN FORM EINER KAUTABLETTE VON DIOSMIN ODER EINER FLAVONOID-EINHEIT ENTHALTEND DIOSMIN

PHARMACEUTICAL COMPOSITION IN THE FORM OF A CHEWABLE TABLET OF DIOSMIN OR OF A FLAVONOID MOIETY COMPRISING DIOSMIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA MD TN**

(30) Priorité: **20.07.2018 FR 1856769**

(43) Date de publication de la demande:
**26.05.2021 Bulletin 2021/21**

(73) Titulaire: **Les Laboratoires Servier**
**92284 Suresnes Cedex (FR)**

(72) Inventeurs:
• **MARSAS, Stéphanie**
  **45430 CHECY (FR)**
• **PEAN, Jean-Manuel**
  **45000 ORLEANS (FR)**

(74) Mandataire: **Giudicelli, Cathy**
**Les Laboratoires Servier**
**Direction Brevets**
**35, rue de Verdun**
**92284 Suresnes Cedex (FR)**

(56) Documents cités:
**EP-A1- 0 541 874**   **EP-A1- 0 711 560**
**EP-A1- 2 745 839**   **FR-A1- 2 661 610**
**FR-A1- 2 845 597**

**Description**

La présente invention est relative à une composition pharmaceutique sous la forme d'un comprimé à croquer fortement dosé en diosmine micronisée. La présente invention concerne également une composition pharmaceutique sous la forme d'un comprimé à croquer fortement dosé de fraction flavonoïque purifiée et micronisée (MPFF).

La fraction flavonoïque est issue d'un extrait de rutacées. La fraction flavonoïque purifiée et micronisée utilisée dans l'invention contient de 87% à 93% de diosmine et d'autres flavonoïdes de manière concomitante. Ces autres flavonoïdes à hauteur environ de 10% comprennent de 2.5% à 5.0% d'hespéridine, de 0.9% à 2.8% d'isorhoifoline, de 0.9% à 2.8% de linarine et moins de 1% de diosmétine.

La diosmine et la fraction flavonoïque selon l'invention sont micronisées. La micronisation du principe actif augmente significativement sa biodisponibilité. La micronisation des flavonoïdes dont la diosmine est particulièrement intéressante pour renforcer l'absorption digestive de ces substances qui ont une faible hydrosolubilité et sont donc peu absorbées par la muqueuse digestive. En conséquence, il est préférable de développer des formes galéniques comprenant de la diosmine ou une fraction flavonoïque micronisée afin d'améliorer la biodisponibilité.

Par ailleurs, la fraction flavonoïque selon l'invention est administrée à des doses journalières allant de 1000mg à 3000mg afin de traiter les manifestations de l'insuffisance veineuse chronique des membres inférieurs. Du fait d'une augmentation des posologies quotidiennes dans le traitement des principales indications thérapeutiques de la fraction flavonoïque et de la diosmine, ces principes actifs doivent être fortement dosés à chacune de leurs administrations. Par ailleurs, les traitements à base de fraction flavonoïque sont des traitements au long cours imposant une prise facile afin de favoriser l'observance du traitement de la part des patients. En conséquence, il est préférable de développer des formes galéniques permettant une prise facile pour les personnes âgées et sans addition d'eau pour les patients ayant une consommation hors foyer.

L'utilisation en thérapeutique de la fraction flavonoïque extraite de rutacées selon l'invention a été décrite dans le brevet EP 0 711 560. Ce brevet décrit une composition de granulés effervescents fortement dosés à 1000mg de fraction flavonoïque. Ces granulés effervescents sont à disperser dans l'eau avant consommation.

Des formes pharmaceutiques de comprimé à croquer, à sucer ou à mâcher contenant de la diosmine ont été décrites dans la demande de brevet internationale WO 2004/032942. Les formes pharmaceutiques selon la demande WO 2004/032942 ne sont pas fortement dosées en diosmine et ne mettent pas en oeuvre du principe actif sous forme micronisée.

Ainsi la présente invention est relative à des compositions pharmaceutiques sous la forme d'un comprimé à croquer fortement dosé en diosmine micronisée ou en fraction flavonoïque purifiée et micronisée (MPFF).

La composition pharmaceutique du comprimé à croquer selon l'invention doit avoir des propriétés fonctionnelles spécifiques afin de répondre aux difficultés technologiques relatives aux comprimés à croquer fortement dosé en principe actif micronisé.

En effet, il est nécessaire que la composition pharmaceutique soit suffisamment compressible en évitant les phénomènes de clivage et de friabilité. La très forte proportion de principe actif et la faible proportion d'excipients, afin de ne pas augmenter la masse finale du comprimé à croquer, ont un impact négatif sur les propriétés mécaniques souhaitées du comprimé à croquer.

Par ailleurs, la micronisation des poudres de principes actifs a pour effet de diminuer la capacité d'écoulement desdites poudres compte-tenu de l'augmentation des forces entre les particules. La taille des particules est un facteur essentiel de la coulabilité des poudres laquelle peut également être influencée par la forme des particules et/ou l'humidité.

L'objet de la présente invention est une composition pharmaceutique sous la forme d'un comprimé à croquer fortement dosé en fraction flavonoïque purifié et micronisé comprenant de la diosmine, de l'hespéridine, de l'isorhoifoline, de la linarine et de la diosmétine.

Par « comprimé à croquer » on entend selon l'invention une forme pharmaceutique connue dans la terminologie anglo-saxonne sous le terme « chewable tablet ». Un comprimé « à croquer » ou un comprimé « à mâcher » désignent ici des formes pharmaceutiques équivalentes.

Les formes pharmaceutiques à croquer sont très bien acceptées par les patients et améliorent leur observance en dépit du fait que ces formes à croquer soient des comprimés volumineux d'au moins 3000mg de masse unitaire.

La diosmine et la fraction flavonoïque sont micronisées dans les compositions pharmaceutiques selon l'invention. La micronisation consiste en un procédé permettant de réduire la taille des particules d'une poudre. La micronisation du principe actif tel que la diosmine ou une fraction flavonoïque peut être réalisée à l'aide de différents systèmes de micronisation. Ces systèmes de micronisation peuvent être un broyeur, un microniseur à jet d'air ou un microniseur à boules pour lesquels la pression des jets d'air ou le débit d'alimentation en poudre varient en fonction de la granulométrie attendue du principe actif micronisé.

La taille des grains est une caractéristique particulièrement importante des matériaux pulvérulents tels que les poudres. La caractérisation de la taille d'une particule est faite en fonction de son diamètre ou le diamètre des sphères équivalentes si la particule est d'une forme irrégulière. Les diamètres de grains déterminés par un granulomètre à diffraction laser permettent de caractériser une distribution granulométrique ou granulométrie $d_{50}$, $d_{90}$ (distribution de la taille des particules).

Dans la présente invention une « $d_{50}$ inférieure à X $\mu$m » signifie qu'au moins 50% des particules du micronisat ont une taille inférieure à X $\mu$m. Dans la présente invention une «$d_{90}$ inférieure à X $\mu$m » signifie qu'au moins 90% des particules du micronisat ont une taille inférieure à X $\mu$m.

Dans un mode de réalisation, la $d_{90}$ du principe actif micronisé est inférieure à 5$\mu$m. Une $d_{90}$ inférieure à 5$\mu$m englobe une $d_{50}$ inférieure à 4$\mu$m, 3$\mu$m, 2$\mu$m, 1.8$\mu$m, 1.6$\mu$m, 1.5$\mu$m.

Dans un mode de réalisation, la $d_{50}$ du principe actif micronisé est inférieure à 5$\mu$m. Une $d_{50}$ inférieure à 5$\mu$m englobe une $d_{50}$ inférieure à 4$\mu$m, 3$\mu$m, 2$\mu$m, 1.8$\mu$m, 1,6$\mu$m, 1,5$\mu$m.

Dans certains modes de réalisations, la diosmine micronisée ou la fraction flavonoïque micronisée et purifiée sont caractérisées par une granulométrie suivante :

- une $d_{50}$ inférieure à 2$\mu$m, de préférence inférieure à 1.6$\mu$m et/ou
- une $d_{90}$ inférieure à 5$\mu$m, de préférence inférieure à 2$\mu$m, de manière encore préférée inférieure à 1.6$\mu$m.

Le diamètre moyen des particules de principe actif micronisé dans la composition pharmaceutique selon l'invention est strictement inférieur à 5$\mu$m, de préférence strictement inférieur 1.6$\mu$m.

Le pourcentage de diosmine micronisée ou de fraction flavonoïque purifiée et micronisée dans la composition pharmaceutique est compris entre 30% et 60% de la masse totale de la composition. Les compositions pharmaceutiques selon l'invention permettent une administration d'une grande quantité de flavonoïdes par voie orale pour chaque dose unitaire. Par compositions pharmaceutiques « fortement dosées » on entend des formulations contenant au moins 20% de principe actif. La quantité de diosmine ou de fraction flavonoïque dans la composition pharmaceutique est comprise 1000mg.

Les compositions pharmaceutiques sous la forme de comprimés à croquer fortement dosés comprennent de 30% à 60% en poids de diosmine micronisée ou de fraction flavonoïque purifiée et micronisée de la masse totale de la composition et de 40% à 70% en poids de polyols de la masse totale de la composition.

Afin de répondre aux contraintes industrielles inhérentes à la fabrication du comprimé à croquer ayant un principe actif micronisé et fortement dosé, il est nécessaire de sélectionner des excipients aidant à surmonter lesdites contraintes industrielles. Un excipient doit s'entendre comme tout composé formant partie de la formulation qui est destiné à agir comme simple support, c'est-à-dire qui n'est pas destiné à avoir une activité biologique. Les compositions pharmaceutiques selon l'invention comprennent au moins un polyol ayant la fonction de diluant.

Un diluant sert à obtenir un volume de poudre suffisant pour fabriquer un comprimé de la taille souhaitée et dont les caractéristiques physiques sont compatibles avec les procédés de fabrication par compression directe par exemple. On utilise comme diluant un ou plusieurs polyols. De préférence le polyol utilisé est le sorbitol. On peut avantageusement employer deux polyols différents et de préférence un mélange de mannitol et sorbitol. Les polyols comme diluant ont l'avantage d'offrir une saveur sucrée et ont d'excellentes propriétés de liant et de compressibilité. Il est possible de substituer au mannitol ou au sorbitol un autre polyol notamment le xylitol ou le maltitol.

Dans la composition pharmaceutique selon l'invention le ratio de la masse du polyol ou des polyols sur la masse de principe actif est strictement inférieur à 2, de préférence le ratio est inférieur à 1.6.

Outre le principe actif micronisé et le ou les polyols, la composition pharmaceutique selon l'invention contient un ou plusieurs excipients pharmaceutiquement acceptables. Par exemple l'objet de l'invention peut être une composition pharmaceutique comprenant le principe actif micronisé, un (des) polyol(s) et un liant ou bien une composition pharmaceutique comprenant le principe actif micronisé, un (des) polyol(s), un liant et un lubrifiant.

Les liants ou agglutinants sont des agents dont le rôle est de lier entre elles les différentes particules de la composition pharmaceutique. Parmi les liants on peut citer la maltodextrine et la povidone. Les lubrifiants ont pour fonction d'éviter les phénomènes de grippage, adhésion, cohésion au cours des différents processus industriels. Le lubrifiant est choisi entre autre parmi l'acide stéarique, le stéarate de magnésium ou le talc.

D'autres excipients pharmaceutiquement acceptables peuvent être ajoutés à la composition pharmaceutique selon l'invention tels que des arômes, des édulcorants ou des liants

A titre d'exemple d'excipients, on peut citer :

Les aromatisants ou arômes, ayant pour objet de masquer les saveurs désagréables et atténuer les consistances terreuses : arôme orange, arôme citron, arôme caramel mou, arôme vanille/citron ;
Les édulcorants augmentent la saveur sucrée de la composition : l'aspartame, l'acésulfame de potassium, la saccharine sodique, le cyclamate de potassium ; et
Les agents d'écoulement comme la silice colloïdale anhydre .

La composition selon l'invention peut être une composition pharmaceutique à libération immédiate, prolongée ou retardée. De préférence la composition selon l'invention est une composition à libération immédiate.

L'invention s'étend au procédé de préparation par granulation humide d'un comprimé à croquer tel que décrit supra, comprenant au moins les étapes suivantes :

a) mélange de diosmine micronisée ou de fraction flavonoïque purifiée et micronisée, de polyols, de liants, d'arômes et d'édulcorants ;
b) après mélange réaliser le mouillage. La masse humide ainsi obtenue étant ensuite granulée, séchée puis calibrée ;
c) lubrification du granulé obtenu à l'étape b) au moyen de silice colloïdale et de stéarate de magnésium ;
d) compression du mélange lubrifié.

Dans un mode de réalisation préféré, le comprimé à croquer selon l'invention est préparé selon un procédé par compression directe comprenant au moins les étapes suivantes :

a) mélange de diosmine micronisée ou de fraction flavonoïque purifiée et micronisée, de polyols, de liants, d'arômes et d'édulcorants ;
b) lubrification du mélange obtenu à l'étape a) au moyen de silice colloïdale et de stéarate de magnésium ;
c) compression directe du mélange lubrifié.

Dans un mode de réalisation également préféré, le comprimé à croquer selon l'invention est préparé selon un procédé par granulation par compactage ou par granulation sèche comprenant au moins les étapes suivantes :

a) mélange de diosmine micronisée ou de fraction flavonoïque purifiée et micronisée, de polyols, de liants, d'arômes et d'édulcorants ;
b) après mélange réaliser le compactage en granulés ;
c) lubrification du granulé obtenu à l'étape b) au moyen de silice colloïdale et de stéarate de magnésium ;
d) compression du mélange lubrifié.

De préférence, on obtient en fin de procédé des comprimés à croquer dont la dureté mesurée par écrasement diamétral est comprise entre 180N à 220N (N=Newtons). De préférence, les comprimés à croquer ont une dureté comprise entre 180N et 200N.

La présente invention concerne également l'utilisation des compositions pharmaceutiques selon l'invention dans le traitement de la maladie veineuse, plus particulièrement de l'insuffisance veineuse tels que jambes lourdes, douleurs, impatiences du primo-décubitus, la fragilité capillaire et le traitement de la crise hémorroïdaire. Ces compositions pharmaceutiques sont utilisées comme veinotonique et vasculo-protecteur.

Les exemples ci-dessous illustrent l'invention de manière non limitative.

Exemple 1 : Compositions pharmaceutiques de comprimés à croquer

| Ingrédients | Quantité (mg) | Teneur (%) |
|---|---|---|
| MPFF | 1000.0 | 33.3 |
| - Diosmine 90% | 900.0 | 30 |
| - Flavonoïdes 10% | 100.0 | 3.3 |
| mannitol | 409.13 | 13.6 |
| Sorbitol | 1227.37 | 40.9 |
| Maltodextrine | 300 | 10 |
| Arôme orange | 33 | 1.1 |
| Acésulfame de potassium | 2 | 0.06 |
| Stéarate de magnésium | 22.5 | 0.75 |
| Silice colloïdale anhydre | 6 | 0.2 |
| Masse finale | 3000.0 | |

| Ingrédients | Quantité (mg) | Teneur (%) |
|---|---|---|
| Diosmine micronisée | 1000.0 | 33.3 |
| mannitol | 409.13 | 13.6 |
| Sorbitol | 1227.37 | 40.9 |
| Maltodextrine | 300 | 10 |
| Arôme orange | 33 | 1.1 |
| Acésulfame de potassium | 2 | 0.06 |
| Stéarate de magnésium | 22.5 | 0.75 |
| Silice colloïdale anhydre | 6 | 0.2 |
| Masse finale | 3000.0 | |

Fabrication des comprimés à croquer de l'exemple 1 :

La diosmine micronisée ou la fraction flavonoïque purifiée et micronisée est soigneusement mélangée aux excipients de la phase interne, i.e. mannitol, sorbitol, maltodextrine, arôme orange, acésulfame de potassium. Le mélange est mouillé avec de l'eau purifiée à l'aide d'une cuve sous pression puis granulé.

Les granulés sont séchés jusqu'à obtenir une humidité résiduelle conforme aux spécifications soit environ 2% d'humidité résiduelle. Les granulés sont ensuite calibrés, homogénéisés puis lubrifiés avec le stéarate de magnésium. Les granulés sont enfin tamisés sur un tamis 0.8mm d'ouverture de maille puis comprimés à l'aide de poinçons.

Les comprimés à croquer de diosmine micronisée et de fraction flavonoïque purifiée et micronisée selon l'exemple 1 ont respectivement une dureté de 196 N et 192 N (N=Newton).

Exemple 2 : propriétés d'écoulement des compositions pharmaceutiques selon l'invention

La coulabilité d'une poudre est son aptitude à s'écouler librement de manière régulière et constante sous forme de particules individuelles. L'aptitude à l'écoulement des poudres conditionne donc les performances et le bon fonctionnement des procédés de production et joue un rôle sur la qualité du produit final. Ainsi, une poudre possédant une bonne coulabilité s'écoule sans aide. À l'opposé, une poudre cohésive a une faible coulabilité et un dispositif mécanique (agitation, vibration) ou chimique (enrobage) doit être prévu pour faciliter sa mise en mouvement.

La coulabilité des poudres et des granulés de diosmine micronisée fortement dosée et de fraction flavonoïque purifiée, micronisée et fortement dosée selon l'invention mesurée par différentes méthodes (indice de Carr, rapport d'Hausner et fonction d'écoulement de Schultze) témoigne que les compositions pharmaceutiques selon l'invention sont très performantes dans les procédés de fabrication des comprimés à croquer. En dépit de facteurs très défavorables au rendement des procédés de fabrication, tels que la micronisation du principe actif et/ou un principe actif fortement dosé, la coulabilité des compositions pharmaceutiques selon l'invention répond aux exigences industrielles et réglementaires.

L'indice de Carr (Ic) qualifie l'écoulement d'un lit de poudre en fonction de la masse volumique. Il est déterminé par l'équation ci-dessous :

$$Ic = (\rho_{tassé}-\rho_{vrac})/\rho_{tassé}$$

où $\rho$ est la masse volumique et Ic une grandeur physique sans dimension. Les grandeurs d'indice de Carr s'interprètent de la manière suivante :

Ic < 0,15 bonne coulabilité
0,15 < Ic < 0,25 coulabilité moyenne
Ic > 0,25 mauvaise coulabilité

Le rapport d'Hausner (Hr) qualifie la compressibilité et l'écoulement d'une poudre à partir de mesures de masse volumique ($\rho$). Il est calculé par le rapport de la masse volumique tassée ($\rho_{tassé}$) sur la masse volumique vrac ($\rho_{vrac}$) selon l'équation: $Hr=\rho_{tassé}/\rho_{vrac}$

Hr est une grandeur physique sans dimension. Si Hr se situe entre 1,0 et 1,2, la poudre est peu compressible, peu cohésive et a un bon écoulement; si Hr se situe entre 1,2 et 1,4, la poudre est compressible, cohésive et présente un mauvais écoulement.

Enfin l'appareil de Schultze est une cellule à cisaillement pour mesurer la fonction d'écoulement (FFC). Un écoulement facile est caractérisé par une FFC comprise entre 4 et 10 et un écoulement libre est caractérisé par une FFC supérieure à 10.

| Méthodes | Poudres ou Granulés | |
|---|---|---|
| | diosmine micronisée | fraction flavonoïque purifiée et micronisée |
| Ic | 0.12-0.25 (coulabilité moyenne à bonne) | |
| Hr | 1.1-1.3 (coulabilité bonne à assez bonne) | |
| FFC | 7-13 (écoulement facile à libre) | |

**Revendications**

1. Composition pharmaceutique sous la forme d'un comprimé à croquer comprenant comme principe actif 1000 mg de diosmine micronisée, **caractérisée en ce que** :

   - la composition comprend au moins un polyol,
   - la composition comprend de 30% à 60% en poids de diosmine micronisée par rapport à la masse totale de la composition.

2. Composition pharmaceutique sous la forme d'un comprimé à croquer comprenant comme principe actif 1000 mg de fraction flavonoïque purifiée et micronisée comprenant de 87% à 93% de diosmine et d'autres flavonoïdes de manière concomitante à hauteur environ de 10% comprenant de 2.5% à 5.0% d'hespéridine, de 0.9% à 2.8% d'isorhoifoline, de 0.9% à 2.8% de linarine et moins de 1% de diosmétine, **caractérisée en ce que** :

   - la fraction flavonoïque est issue d'un extrait de rutacées,
   - la composition comprend au moins un polyol,
   - la composition comprend de 30% à 60% en poids de fraction flavonoïque purifiée et micronisée par rapport à la masse totale de la composition.

3. Composition pharmaceutique selon l'une quelconque des revendications 1 ou 2, **caractérisée en ce qu'**elle comprend de 40% à 70% en poids de polyols de la masse totale de la composition.

4. Composition pharmaceutique selon l'une quelconque des revendications 1 à 3, **caractérisée en ce que** le ratio de la masse du polyol ou des polyols sur la masse de principe actif est strictement inférieur à 2.

5. Composition pharmaceutique selon l'une quelconque des revendications 1 à 4, dans laquelle un polyol est le sorbitol.

6. Composition pharmaceutique selon l'une quelconque des revendications 1 à 5, comprenant un polyol et un liant.

7. Composition pharmaceutique selon l'une quelconque des revendications 1 à 6, comprenant un polyol, un liant et un lubrifiant.

8. Procédé de fabrication d'une composition pharmaceutique selon l'une quelconque des revendications 1 à 7, **caractérisé en ce qu'**il s'agit d'un procédé par granulation humide, granulation sèche ou compression directe.

9. Composition pharmaceutique selon l'une quelconque des revendications 1 à 7 pour son utilisation dans le traitement de l'insuffisance veineuse et de la crise hémorroïdaire.

**Patentansprüche**

1. Pharmazeutische Zusammensetzung in Form einer Kautablette, umfassend als Wirkstoff 1000 mg mikronisiertes Diosmin, **dadurch gekennzeichnet, dass**

   - die Zusammensetzung mindestens ein Polyol umfasst,
   - die Zusammensetzung 30 bis 60 Gew.-% mikronisiertes Diosmin, bezogen auf die Gesamtmasse der Zusammensetzung, umfasst.

2. Pharmazeutische Zusammensetzung in Form einer Kautablette, umfassend als Wirkstoff 1000 mg einer gereinigten und mikronisierten Flavonoidfraktion, umfassend 87% bis 93% Diosmin und gleichzeitig andere Flavonoide in einer Höhe von etwa 10%, umfassend 2,5% bis 5,0% Hesperidin, 0,9% bis 2,8% Isorhoifolin, 0,9% bis 2,8% Linarin und weniger als 1% Diosmetin, **dadurch gekennzeichnet, dass**:

   - die Flavonoidfraktion aus einem Rutaceae-Extrakt stammt,
   - die Zusammensetzung mindestens ein Polyol umfasst,
   - die Zusammensetzung 30 bis 60 Gew.-% einer gereinigten und mikronisierten Flavonoidfraktion, bezogen auf die Gesamtmasse der Zusammensetzung, umfasst.

3. Pharmazeutische Zusammensetzung nach einem der Ansprüche 1 oder 2, **dadurch gekennzeichnet, dass** sie 40 bis 70 Gew.-% Polyole, bezogen auf die Gesamtmasse der Zusammensetzung, umfasst.

4. Pharmazeutische Zusammensetzung nach einem der Ansprüche 1 bis 3, **dadurch gekennzeichnet, dass** das Verhältnis der Masse des Polyols oder der Polyole zur Masse des Wirkstoffs unbedingt weniger als 2 beträgt.

5. Pharmazeutische Zusammensetzung nach einem der Ansprüche 1 bis 4, in der ein Polyol Sorbitol ist.

6. Pharmazeutische Zusammensetzung nach einem der Ansprüche 1 bis 5, umfassend ein Polyol und ein Bindemittel.

7. Pharmazeutische Zusammensetzung nach einem der Ansprüche 1 bis 6, umfassend ein Polyol, ein Bindemittel und ein Gleitmittel.

8. Verfahren zur Herstellung einer pharmazeutischen Zusammensetzung nach einem der Ansprüche 1 bis 7, **dadurch gekennzeichnet, dass** es sich um ein Verfahren durch Nassgranulation, Trockengranulation oder Direktkompression handelt.

9. Pharmazeutische Zusammensetzung nach einem der Ansprüche 1 bis 7 zur Verwendung bei der Behandlung von Veneninsuffizienz und Hämorrhoidalkrise.

**Claims**

1. A pharmaceutical composition in the form of a chewable tablet comprising as active ingredient 1000 mg of micronized diosmin,
   **characterized in that**:

   - the composition comprises at least one polyol,
   - the composition comprises from 30% to 60% by weight of micronized diosmin relative to the total mass of the composition.

2. A pharmaceutical composition in the form of a chewable tablet comprising as active ingredient 1000 mg of purified and micronized flavonoid moiety concomitantly comprising from 87% to 93% of diosmin and up to approximately 10% of other flavonoids comprising from 2.5% to 5.0% of hesperidin, from 0.9% to 2.8% of isorhoifolin, from 0.9% to 2.8% of linarin and less than 1% of diosmetin, **characterized in that**:

   - the flavonoid moiety is derived from a rutaceae extract,
   - the composition comprises at least one polyol,
   - the composition comprises from 30% to 60% by weight of purified and micronized flavonoid moiety relative to the total mass of the composition.

3. The pharmaceutical composition according to any of claims 1 or 2, **characterized in that** it comprises from 40% to 70% by weight of polyols relative to the total mass of the composition.

4. The pharmaceutical composition according to any of claims 1 to 3, **characterized in that** the ratio of the mass of the at least one polyol to the mass of active ingredient is strictly less than 2.

5. The pharmaceutical composition according to any of claims 1 to 4, wherein a polyol is sorbitol.

6. The pharmaceutical composition according to any of claims 1 to 5, comprising a polyol and a binder.

7. The pharmaceutical composition according to any of claims 1 to 6, comprising a polyol, a binder and a lubricant.

8. A process for manufacturing a pharmaceutical composition according to any of claims 1 to 7, **characterized in that** it is a process by wet granulation, dry granulation or direct compression.

9. The pharmaceutical composition according to any of claims 1 to 7 for use in the treatment of venous insufficiency and hemorrhoidal crisis.

**EP 3 823 588 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0711560 A
- WO 2004032942 A